# EUROPEAN PATENT APPLICATION

(11) **EP 4 850 663 A1**
(43) Date of publication of application: **09.12.2026**
(21) Application number: 25305803.6
(22) Date of filing: 03.06.2025
(51) Int. Cl.: A61K 8/60, A61K 8/9789, A61Q 19/08

(54) **COMPOSITION COMPRISING LEONURUS JAPONICUS ROOT EXTRACT AND USES THEREOF**

(71) Applicant: Clariant International Ltd., 4132 Muttenz (CH); Plant Advanced Technologies PAT SA, 54500 Vandoeuvre-lès-Nancy (FR)
(72) Inventor: The designation of the inventor has not yet been filed
(74) Representative: Cabinet Becker et Associés

(57) **Abstract**

The present invention refers to a root extract of the plant *Leonurus japonicus* and use thereof in cosmetics. The present invention also relates to a root extract of *Leonurus japonicus* enriched in phenylpropanoid glycosides.

## Description

### Field of the invention

The present invention refers to a root extract of the plant *Leonurus japonicus* and use thereof in cosmetics. The present invention also relates to a root extract of *Leonurus japonicus* enriched in phenylpropanoid glycosides. Furthermore, the present invention refers to a cosmetic ingredient comprising such root extract in a cosmetically acceptable vehicle.

### Background of the invention

The skin is one of the largest organs in the human body and performs functions that are crucial to maintain our physiology, such as regulating body temperature, preventing dehydration and providing sensory perception. It also forms a protective barrier against environmental stresses thanks to its complex organization, divided into three distinct layers: epidermidis, dermis, hypodermis and crisscrossed by vascular network and nerve fibers.

Skin aging is a multi-factorial process driven by both intrinsic (e.g., time, genetic factors, hormones) and extrinsic (e.g., UV-exposure, pollution) factors. Characteristics of aging include visible signs such as thin and dry skin, fine wrinkles, decreased elasticity, aberrant pigmentation. Among which epidermal thinning causes, a decrease of proliferation and renewal capacity of basal keratinocytes is currently reported. During aging, the extracellular matrix undergoes structural alterations and degradation, leading to the increase of wrinkles and loss of elasticity of the skin.

One of the key parameters in chronological ageing for women is the loss of hormones occurring at a certain period of life. This phenomenon, called menopause, can be defined, clinically, as the period of time in a woman's life after twelve consecutive months of amenorrhea and in most women, menopause is preceded by a period (perimenopause) during which many endocrine, biological and clinical changes are occurring (Reus et al., 2020). Particularly, the decline in hormones levels from several years before - perimenopause - and during menopause has a strong impact on skin quality visual aspect and comfort. Indeed, the skin is an endocrine organ and a major target of hormones such as estrogens, androgens, and cortisol. It is known that, in early menopause, skin collagen levels decrease rapidly with a collagen reduction of approximately 30% in the first 5 years, followed by a further decline of 2% per year for the next 15 years (Zouboulis et al., 2022). In addition to a decrease in collagen content, these hormones change also leads to a decrease in elasticity and water content, skin thickening, vascular fragility, impacting on all skin layer quality (Lephart et al., 2022). Besides, the decline in estrogen reduces sebaceous gland activity and lipid synthesis, leading to decreased sebum levels and exacerbated skin dryness. Indeed, sky dryness is one of the most frequent effects of hormonal drop experimented by women in perimenopause or in menopause. Skin dryness and dehydration caused by hormonal change impair the visual aspect of the skin but also the comfort of the skin. Women in perimenopause or menopause can experiment a skin discomfort associated with unpleasant sensations such as tightness, tingling, transient redness and transient flush. There is still an unmet need in providing efficient and specific solutions for women undergoing this specific period of life and enabling to alleviate the symptoms of menopause. Oral solutions exist to replace the hormonal decrease, but it is still temporary solution and often proposed to young menopausal women or women with high side effects of menopause. As the skin is highly impacted by menopause, there is a need for women to preserve their skin quality during this period. Some cosmetic products are available on the market to accompany women in this period, but active ingredients specifically dedicated for menopausal women are still a need on the market. Indeed, currently on the market, the range cosmetics products aimed at menopausal women are mainly anti-ageing or moisturizing ingredients, using conventional anti-aging ingredient such as retinol, ascorbic acid but no specific active ingredient targeting the drop in hormones that occurs at menopause inducing a drastic impact on skin quality. Thus, the desire for active ingredients addressing all the hallmarks of hormonal aging, to help women going through this period, by improving their skin quality and consequently their quality of life, is growing (Flament et al., 2023).

There is thus a need for cosmetic active ingredient able to counteract the effects of hormonal changes in the skin in perimenopausal or menopausal women.

### Summary of the invention

It has been surprisingly discovered that an extract of the root of *Leonurus japonicus* may be of interest in cosmetics, preferably for ameliorating and/or maintaining the quality of a mature skin e.g., the skin of a woman of at least 40 years old and/or in perimenopause or menopause.

Therefore, an aspect of the present invention pertains to the (cosmetic) use of an alcoholic or a hydroalcoholic root extract of *Leonurus japonicus,* as an active ingredient, for ameliorating and/or maintaining the quality and/or the comfort of a mature skin. Another aspect of the present invention refers to a root extract of *Leonurus japonicus* comprising phenylpropanoid glycosides selected from the group consisting of lavandulifolioside, verbascoside, leonoside A, leucosceptoside A and leonoside B, wherein the lavandulifolioside represents 5 to 30% by weight relative to the total weight of the dry extract.

A further aspect of the invention relates to a cosmetic ingredient comprising (or consisting of) a root extract of *Leonurus japonicus* comprising phenylpropanoid glycosides according to an aspect above, as an active ingredient, and a cosmetically acceptable vehicle.

A yet further aspect of the invention pertains to a cosmetic composition comprising a root extract of *Leonurus japonicus* according to the above aspect, or comprising a cosmetic ingredient comprising root extract *of Leonurus japonicus* according to the above aspect, and optionally one or more excipients, other than root extract of *Leonurus japonicus,* which are preferably cosmetically acceptable, wherein the composition is a composition for topical use selected from the group consisting of a solution, a suspension, an emulsion, a cream, a paste, a gel, a lotion, a powder, a soap, a surfactant-containing water, an oil, a shampoo, and a spray.

A yet further aspect of the invention relates to a nutraceutical composition comprising root extract of *Leonurus japonicus* according to the above aspect, and optionally one or more excipients nutraceutically acceptable, wherein the composition is a composition for oral use.

The present invention also refers to a cosmetic non-therapeutic method for ameliorating and/or maintaining the quality and/or the comfort of a mature skin, the method comprising a topical application of an effective amount of an alcoholic and/or a hydroalcoholic root extract of *Leonurus japonicus* according to the above aspect, or a cosmetic ingredient according to the above aspect on the part of the body in need.

All documents cited or referenced herein ("herein cited documents") together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

The elements of the present invention will be described in more detail here below. These elements are listed with specific embodiments; however, they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise. Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

Unless defined otherwise, all technical and scientific terms used herein have the same meaning as commonly understood by a skilled person. Although the methods and materials described herein are preferred, other methods and materials similar or equivalent to those described herein can be used in the practice or testing of the present invention as well.

### Description of figures

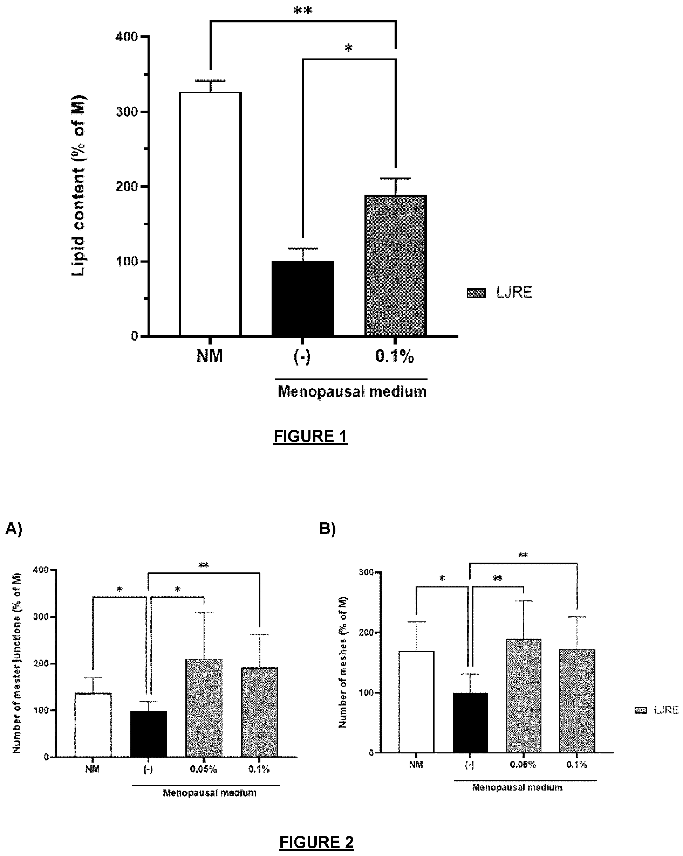

**Figure** 1 shows the production of lipid droplets by human sebocytes treated or not 48h with the root extract of *Leonurus japonicus* ("LJRE") of the present invention under menopausal stress was measured by immunofluorescence. Results are expressed in percentage of variation compared to control condition. NM : human sebocytes in normal medium, (-) : untreated human sebocytes in menopausal medium, and 0.1% : human sebocytes in menopausal medium treated with 0.1% of LJRE. Statistics: ANOVA test *versus* control, * p<0.05, ** p<0.01, *** p>0.001
**Figure** 2 shows the formation of microvascular network by menopause-stressed human umbilical vein endothelial cells (HUVEC) treated or not 16h with the root extract of *Leonurus japonicus* ("LJRE") of the present invention at 0.05% and 0.1%, that was assessed by image analysis of the network on key parameters: number of master junctions (A) and number of meshes (B) and compared with the non-menopausal (NM) level. Results are expressed in percentage of variation compared to menopausal (M) control condition. Statistics: ANOVA test *versus* control, * p<0.05, ** p<0.01, *** p>0.001.

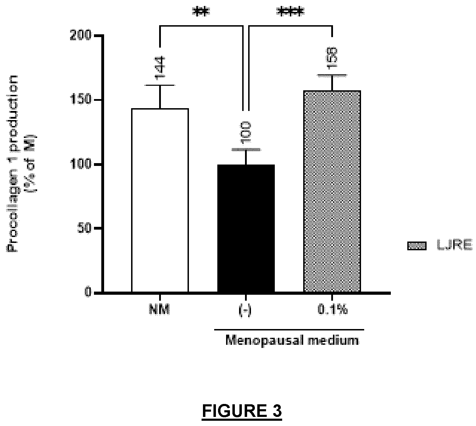

**Figure 3** demonstrates the production of pro-collagen I of menopause-stressed human dermo-fibroblasts (NHDF) treated or not 72h with the root extract of *Leonurus japonicus* ("LJRE") of the present invention at 0.1%, that was measured by ELISA and compared to non-menopausal (NM) level. Results are expressed in percentage of variation compared to menopausal control condition. Statistics: ANOVA test *versus* control, * p<0.05, ** p<0.01, *** p>0.001.

### Detailed description of the invention

*Leonurus japonicus,* also called the Chinese motherwort, is an annual or biennial herbaceous flowering plant of the *Lamiaceae* family. *Leonurus japonicus* is widely used in traditional medicine in China, Japan and Korea. Its Chinese name is *Yi Mu Cao,* which means "good for women" and indicates its traditional function in gynecologic diseases. The aerial parts of *Leonurus japonicus* are commonly used in Chinese herbal medicine to treat menstrual disturbance, dysmenorrhea, amenorrhea, during labor or to prevent postpartum hemorrhage.

The prior art describes that extracts from the aerial parts of *Leonurus japonicus* can be used in cosmetic products. For example, CN-A 108451801 discloses motherwort leaf and/or stem extract as one of several raw materials entering in the composition of a shower cream. WO-A 2023030041 discloses a hydroalcoholic extract of the aerial parts *Leonurus japonicus* for promoting the synthesis of type I collagen, thus for increasing the skin elasticity.

To the best knowledge of the Applicant, the prior art would not specifically describe or suggest the use of a root extract of *Leonurus japonicus* as a cosmetic active ingredient, let alone for the management of mature skin.

An aspect of the present invention relates to the (cosmetic) use of an alcoholic or hydroalcoholic root extract of *Leonurus japonicus,* as an active ingredient, for ameliorating and/or maintaining the appearance and/or the quality and/or the comfort of a mature skin.

The Applicant surprisingly showed that a root extract of *Leonurus japonicus* can counteract the effects of hormone decline due to age in the skin. Hormonal, and in particular estrogen, decrease associated with perimenopause or menopause impacts the visual aspect and the comfort of the skin. Estrogen decline results in a decrease in both collagen synthesis and sebum production, and can impair the skin microvascular network. As illustrated in Example 4, the Applicant showed that a root extract of *Leonurus japonicus* is able to prevent or treat the effects of hormonal changes in the skin. Such effects were evidenced *in vitro* by using a medium mimicking menopausal changes. As shown in Figure 1, lipid production dramatically decreases in sebocytes incubated in menopausal medium as compared to basal, non-menopausal, condition. This decrease is significantly counteracted by the root extract of *Leonurus japonicus* ("LJRE") according to the invention.

The Applicant further showed that LJRE is able to restore the master junctions and mesh numbers in HUVECs incubated in menopausal conditions to levels comparable with the non-menopausal condition. Accordingly, LJRE can restore the complexity and density of the microvascular network in mature skin (Figure 2).

The Applicant also showed that LJRE is able to restore the production of pro-collagen in NHDF cultured under menopausal conditions to levels similar to those observed in non-menopausal conditions (Figure 3).

Such results demonstrate that the root extract of *Leonurus japonicus* of the invention can exert pleiotropic actions able to prevent or correct the global effects caused by hormonal decline in the skin.

The Applicant also showed that LJRE has a composition distinct from that of extracts obtained from aerial parts of *Leonurus japonicus.* The LJRE of the invention is characterized by the presence of specific phenylpropanoid glycosides ("PPGs") among which lavandulifolioside.

Thus, the invention relates to a root extract of *Leonurus japonicus* as described herein and to its use as a cosmetic active agent, in particular for the management of a mature skin and/or for preventing, delaying the onset, decreasing or alleviating the physiological alterations due to hormonal decline in the skin of a woman of at least 40 years old, preferably in perimenopause or menopause.

### - Leonurus japonicus root extract of the invention

In a first aspect, the invention relates to a root extract of *Leonurus japonicus.*

A root extract of *Leonurus japonicus* (also called extract of the root of *Leonurus japonicus* or *Leonurus japonicus* root extract) refers to any extracts obtained from roots by any methods known in the art such as distillation, extraction with sub-critical or supercritical fluid such as supercritical CO₂ or subcritical water, solid-liquid extraction e.g. by maceration, decoction, dipping and the like, of the roots, in an appropriate solvent.

In the context of the invention, the extract of *Leonurus japonicus* (also interchangeably called "extract" or "root extract") is preferably obtained by extraction of the roots with a hydrophilic solvent. The hydrophilic solvent is preferably an alcoholic or hydroalcoholic solvent.

As used herein, a hydroalcoholic solvent (or mixture) is meant a solvent mixture which essentially consists or consists of one or more alcohols and water. As used herein, "hydroalcoholic solvent consisting essentially of one or more alcohols and water" means that said water and one or more alcohols together account for more than 95%, preferably for more than 96%, 97%, 98%, 99% or 99.5% by weight relative to the total weight of the solvent.

A preferred hydroalcoholic solvent refers to a mixture alcohol/water wherein the alcohol accounts for more than 50%, preferably more than 60%, 70%, 80%, 90% or 95% by weight of the total weight of the solvent.

The water can be mineral water, deionized water, or purified water. In some embodiments, one or more aqueous buffers may be present in the hydroalcoholic solvent.

As used herein, the alcohol may be a lower alcohol, a glycol (also called diol or alkanediol), glycerol or a combination thereof.

Lower alcohols (typically C₂-C₅ alcohols) encompass, without being limited to, methanol, ethanol, propanol, butanol, pentanol, and isomers thereof such as isopropanol, isobutanol or tert-butanol.

Glycols encompass, without being limited to C₂-C₅ alkanediols such as dipropylene glycol, propane-1,3-diol, propane-1,2-diol, pentylene glycol, butane-1,3-diol or a combination thereof.

In some embodiments, a pH adjusting agent such as acid citric or phytic acid is added in the extraction solvent, e.g. at an amount of below 5% by weight, e.g. below 1% by weight. In a particular embodiment, the root extract of *Leonurus Japonicus* is obtained by extraction with a solvent selected from the group consisting of glycerol, propane-1,2-diol, pentylene glycol, butane-1,3-diol, propane-1,3-diol, mixtures thereof with water and combinations thereof, preferably from propane-1,3-diol, glycerol, or a mixture thereof with water and combinations thereof.

In some embodiment, the extraction solvent is propane-1,3-diol or a propane-1,3-diol/water mixture, preferably with a weight ratio higher than 2/1, such as higher than 4/1, 9/1 or 95/5.

The extract of the invention can be obtained for instance from fresh, lyophilized, frozen or dried roots. Preferably the extract is obtained from dried roots.

In some embodiment, the roots can be cut from the plant.

In some embodiment, the plant of *Leonurus Japonicus* is cultured soilless, e.g. aeroponically.

Soilless culture such as aeroponic culture may enable a maceration of the roots without cutting them from the plant and without impairing the viability of the plant. This particular embodiment is detailed further below.

Typically, the roots of *Leonurus japonicus* are macerated in a solvent as described above during a time sufficient to obtain an effective extraction of the molecules of interest (e.g. from few minutes such as 5, 10 or 30 min to several hours such as 2, 5, 10, 15, 20, 24 or 30h). The extraction may be performed at room temperature (e.g. when the roots are not cut from the plant) or under heating e.g. from 35°C to 80°C preferably from 40°C to 60°C, in particular from 45° to 55°C.The liquid phase is then recovered so as to obtain the extract. Such a liquid is optionally, at least partially, evaporated. The extract of the invention can be recovered in liquid form, in a sticky form, or in a solid form e.g. in a powder form.

One or several steps can be performed prior to, or after, the extraction step.

For instance, the roots can be subjected to one or more treatments before being macerated such as a step of washing, cutting, drying including freeze-drying or heat-drying, grinding, cryo-grinding and combinations thereof.

For illustration purpose only, the resulting extract can be diluted in an appropriate vehicle (hereafter dilution solvent), absorbed on an appropriate support (e.g. maltodextrin) or formulated, e.g. into a cosmetic composition. Appropriate dilutions solvents and cosmetic compositions are described further below.

As illustrated in the Example section, the Applicant surprisingly showed that a root extract of *Leonurus japonicus* obtained with a hydrophilic solvent can contain significant amounts of phenylpropanoid glycosides (PPGs). Such PPGs were not found in extracts obtained from aerial parts of the plant. Besides, the Applicant surprisingly showed that a root extract of *Leonurus japonicus* obtained with a hydrophilic solvent such as propanediol has a significant content of lavandulifolioside which appears to be the prevalent phenylpropanoid glycosides found in root extracts.

To the knowledge of the Applicant, lavandulifolioside has never been detected in *Leonurus japonicus,* let alone in *Leonurus japonicus* roots. The absence of lavandulifolioside in aerial part sample is indeed described as a marker that enables to distinguish *Leonurus japonicus* from other species such as *Leonurus cardiaca* and *Leonurus sibiricus* (Kuchta et al., Nat. Prod Commun, 11(8), 1129-1133, 2016). Without wishing to be bound by any theory, the Applicant is of the opinion that phenylpropanoid glycosides, including lavandulifolioside, contribute, at least in part, in the biological effects of the extract according to the invention.

In a particular aspect, the invention relates to a root extract of *Leonurus japonicus,* preferably an alcoholic or hydroalcoholic root extract of *Leonurus japonicus* which comprises a lavandulifolioside.

In some embodiments, the lavandulifolioside accounts for at least 55% by weight, e.g. for at least 60%, 65%, 70%, 75%, 80% or 85% by weight of the total weight of the phenylpropanoid glycosides present in the root extract.

In a particular aspect, the invention relates to a root extract of *Leonurus japonicus,* preferably an alcoholic or hydroalcoholic root extract of *Leonurus japonicus* which comprises phenylpropanoid glycosides selected from lavandulifolioside, verbascoside, leonoside A, leucosceptoside A, leonoside B, and combinations thereof.

In a particular embodiment, verbascoside, leonoside A, leucosceptoside A and leonoside B together account for less than 40%, e.g. for less than 35%, 30%, 25%, 20% or 15% by weight of the total weight of the PPGs present in the root extract.

In a particular embodiment, the root extract of *Leonurus japonicus* has a content of PPGs of at least 2%, preferably of at least 2.5%, 3.0%, 3.5%, 4.0%, 5.0%, 6.0% or 7% by weight relative to the total weight of the dry extract.

In a particular embodiment, the root extract of *Leonurus japonicus* has a content of PPGs of at least 5%, preferably of at least 7% by weight relative to the total weight of the dry extract, wherein the lavandulifolioside accounts for at least 60%, preferably at least 70%, or 75%, or 80% by weight of the total weight of the phenylpropanoid glycosides present in the root extract.

The Applicant showed that it is possible to increase the global content of phenylpropanoid glycosides in the root extract, e.g. by culturing the plants in soilless conditions. In particular, it is possible to significantly increase the content in lavandulifolioside in the root extract by controlling the culture conditions.

For instance, the phenylpropanoid glycosides content in the roots can be increased by submitting the plant to a stimulation step. For instance, a stimulation step may be a step of repeatedly subjecting the plant to a nitrogen-deficient nutrient solution e.g. during one to four weeks. Such a stimulation step is described further below.

In some embodiment, the root extract of *Leonurus japonicus* has a content in phenylpropanoid glycosides of at least 5%, e.g. from 6.0% to 40% by weight relative to the total weight of the dry extract.

According to an embodiment, the lavandulifolioside represents 5 to 30% by weight, preferably 6 to 30% by weight, even preferably 6 to 25% by weight, more preferably 7 to 25% by weight, in particular 7 to 20% by weight relative to the total weight of the dry extract.

According to an embodiment, lavandulifolioside, verbascoside, leonoside A, leucosceptoside A and leonoside B represent together at least 7.5%, more preferably at least 10% by weight relative to the total weight of the dry extract. Advantageously, the phenylpropanoid glycosides represent 7.5 to 36% by weight, preferably 8 to 30% by weight, more preferably 10 to 30% by weight, even more preferably 12 to 30% by weight, in particular 12 to 25% by weight, relative to the total weight of the dry extract.

Another aspect of the present invention refers to a root extract of *Leonurus japonicus* comprising phenylpropanoid glycosides selected from the group consisting of lavandulifolioside, verbascoside, leonoside A, leucosceptoside A and leonoside **B,** wherein the lavandulifolioside represents 5 to 30% by weight relative to the total weight of the dry extract.

According to an embodiment, the lavandulifolioside represents at least 6% by weight, more preferably at least 7% by weight relative to the total weight of the dry extract.

According to an embodiment, the verbascoside represents at least 0.2% by weight, preferably 0.3% by weight, more preferably at least 0.4% by weight relative to the total weight of the dry extract. Advantageously, the verbascoside represents 0.2% to 3% by weight, preferably 0.3% to 3% by weight, more preferably 0.4% to 2% by weight, even more preferably 0.4% to 1.5% by weight, in particular 0.4% to 1% by weight, relative to the total weight of the dry extract.

According to an embodiment, the leonoside A represents at least 0.3% by weight, more preferably at least 0.6% by weight relative to the total weight of the dry extract. Advantageously, the leonoside A represents 0.3% to 2.5% by weight, preferably 0.4% to 2.5% by weight, more preferably 0.4% to 2% by weight, in particular 0.4% to 1.5% by weight, relative to the total weight of the dry extract.

According to an embodiment, the leucosceptoside A represents at least 0.02% by weight, more preferably at least 0.04% by weight relative to the total weight of the dry extract. Advantageously, the leucosceptoside A represents 0.03% to 0,35% by weight, preferably 0.03% to 0.3% by weight, more preferably 0.04% to 0.25% by weight, even more preferably 0.04% to 0.2% by weight, in particular 0.04% to 0.15% by weight, relative to the total weight of the dry extract.

According to an embodiment, the leonoside B represents at least 0.2% by weight, more preferably at least 0.4% by weight relative to the total weight of the dry extract. Advantageously, the leonoside B represents 0.2 to 1.5% by weight, preferably 0.2 to 1.2% by weight, more preferably 0.3 to 1.2% by weight, in particular 0.3 to 1% by weight, relative to the total weight of the dry extract.

According to an embodiment, the phenylpropanoid glycosides represent together at least 7.5%, more preferably at least 10% by weight relative to the total weight of the dry extract. Advantageously, the phenylpropanoid glycosides represent 7.5% to 36% by weight, preferably 8% to 30% by weight, more preferably 10% to 30% by weight, even more preferably 12% to 30% by weight, in particular 12% to 25% by weight, relative to the total weight of the dry extract.

In some embodiment, the extract of root of *Leonurus japonicus* is characterized in that:
- the phenylpropanoid glycosides account for 6.0% to 40.0%, e.g. from 7.0% to 36.0%, by weight relative to the total weight of the dry extract, wherein the phenylpropanoid glycosides is the sum of lavandulifolioside, verbascoside, leonoside A, leucosceptoside A and leonoside B,
- lavandulifolioside accounts for 5.0% to 30.0% e.g. from 6.0% to 27.0% by weight relative to the total weight of the dry extract,
- verbascoside accounts for less than 5.0 %, e.g. from 0.1% to 4.0% by weight relative to the total weight of the dry extract,
- leonoside A accounts for less than 4.0 %, e.g. from 0.1% to 3.5% by weight relative to the total weight of the dry extract,
- leucosceptoside A accounts for less than 2.0%, e.g. from 0.01% to 0.5% by weight relative to the total weight of the dry extract, and
- leonoside B accounts for less than 3.0%, e.g. from 0.01% to 2.0 % by weight relative to the total weight of the dry extract.

According to an embodiment, the root extract of *Leonurus japonicus* may further contain one or more iridoids glycosides selected from ajugoside (CAS 52916-96-8), or 8-O-acetylharpagide (CAS 6926-14-3), or a combination thereof.

According to an embodiment, the root extract may further contain one or more fatty acids.
By "lavandulifolioside" is meant a compound corresponding to the CAS 117895-00-8
By "verbascoside" is meant a compound corresponding to the CAS 61276-17-3
By "leonoside A" is meant a compound corresponding to the CAS 140147-66-6
By "leonoside B" is meant a compound corresponding to the CAS 140147-67-7
By "leucosceptoside A" is meant a compound corresponding to the CAS 83529-62-8
By "phenylpropanoid glycosides" or "PPGs" is preferably meant the sum of lavandulifolioside, verbascoside, leonoside A, leucosceptoside A and leonoside B.

The Applicant surprisingly showed that that a root extract of *Leonurus japonicus* may be enriched in compounds of interest, by culturing said plant under certain conditions without compromising the overall plant development. These conditions include culturing the plant under soilless conditions e.g. aeroponically.

In some embodiment, the root extract of *Leonurus japonicus* is enriched in phenylpropanoid glycosides.

By "root enriched in phenylpropanoid glycosides", "root enriched in PPGs" and "enriched root" are meant a root extract of said plant containing a higher amount (e.g. of at least 1.1, 1.2, 1.5, 2.0, 3.0 or more -fold) of at least one of lavandulifolioside, verbascoside, leonoside A, leucosceptoside A and leonoside B, in comparison to a root extract of the same plant cultured in soil in standard conditions.

Typically, such an extract may have a content in phenylpropanoid glycosides of at least 5% by weight relative to the total weight of the dry extract.

In a particular embodiment, the root extract of *Leonurus japonicus* is obtained from a plant cultured under soilless conditions, in particular aeroponically.

The soilless conditions of culture enable to increase the phenylpropanoid glycosides content in the roots of *Leonurus japonicus* but also enable to have access to the roots of without the destruction of all or part of the plant. Such soilless is thus consistent with the Nagoya Protocol and the International Standard (ISO 26000) guidelines in order to control the access to biodiversity and for ensuring sustainable development and social responsibility.

When the roots are considered sufficiently developed, they can be contacted with an extraction solvent by immersion, dipping or preferably by maceration. The roots release the compounds of interest, e.g. metabolites including PPGs, directly in the extraction solvent. In some embodiment, the roots may be cut from the plant, without total loss of viability of the plant that can be returned for culturing and then reused. The roots may be dried before being macerated. Alternatively, the roots can be macerated without being separated from the plant.

The above method is adapted from the method described in the document WO-A 2001/33942. The teaching of this document is incorporated by reference in the specification of the present invention.

Prior to the maceration step, the roots can be subjected to a stimulation step so as to promote the subsequent release of the molecules of interest (e.g. the PPGs and other metabolites) in the extraction solvent.

The stimulation step can be performed by contacting the roots with a nutrient solution, preferably with a nitrogen-deficient solution, e.g. by pulverization, spraying or dipping. Such a step can be repeated several times, e.g. during one week to four weeks. Accordingly, an example of a suitable method for the preparation of the root extract of the plant *Leonurus japonicus* according to the present invention may comprise a step of cultivating the plant in soilless conditions, preferably aeroponically, a step of stimulating of said roots by means of a nutrient solution, a step of solid/liquid extraction by means of maceration solvent of the stimulated roots, a step of recovering the extract, and optionally a step of diluting the extract in a dilution solvent.

Advantageously, the nutrient solution suitable for the stimulation step comprises essential mineral salts - nitrogen (N), phosphorus (P) and potassium (K) - wherein said nutrient solution may be a nitrogen-deficient solution having a nitrogen proportion less than the nitrogen proportion usually considered as optimal for plant growth and in particular root growth. A skilled artisan would be capable of determining the proportions of N-P-K to be used for obtaining a maximum growth of the plant.

As an alternative embodiment, the roots can be cut from the plant cultured in soilless conditions before being extracted (e.g., by maceration) with the extraction solvent. Preferably, the extraction is performed on dried roots.

It is to be noticed that the method of preparation of the root extract of *Leonurus japonicus* according to the present invention should not be interpreted as being limited to the above culturing method, namely aeroponic cultivation.

In the context of the invention, a plant is cultivated in soilless conditions means that the development of the plant roots does not take place in soil, for example, the roots can develop in a reconstituted medium.

By "aeroponically" is meant a mode of soilless cultivation for which the roots of the plants are not in permanent contact neither with a solid medium nor with a liquid nutrient medium. "Aeroponic culture" encompasses cultivating plants in air or mist environment without using any soil, aggregate or liquid substrate, while spraying stems and roots with a nutrient solution.

By "root maceration in solvent" is meant a root maceration for which the solvent placed in contact with the roots is an extraction solvent which can be, for example lower alcohols, glycols, glycerol, or an hydroalcoholic mixture thereof. Lower alcohols may be preferably selected from the group of methanol, ethanol, propanol, isoopropanol or a combination thereof. Glycols may be preferably selected from dipropylene glycol, propane-1,3-diol, propane-1,2-diol, pentylene glycol, butane-1,3-diol, or a combination of two or more thereof. Preferably, glycol may be propane-1,3-diol.

Typically, the solvent in the form of hydroalcoholic may comprise between 0.01% to 99.99% by weight, e.g. from 10% to 99%, from 30% to 99% or from 50% to 99%, preferably from 50% to 95% by weight of alcohol (namely lower alcohol, glycol and/or glycerol as described herein) by weight relative to the total weight of the mixture. In particular, the maceration solvent may be a hydroalcoholic solvent comprising from 50% to 85% of pentylene glycol, or comprising from 50% to 85% of propane-1,3-diol, preferably bio-based propane-1,3-diol.

By "nutrient solution" is meant an aqueous solution comprising nitrogen, phosphorus and potassium in an optimal amount and in an optimal ratio each other for obtaining a maximum root growth.

By "nitrogen-deficient nutrient solution" is meant a nutrient solution comprising less than 15% by weight, preferably less than 10%, even preferably less than 5%, in particular 0% of nitrogen. Typically, the nitrogen-deficient nutrient solution allows the obtention of a maximum amount of PPGs, without impairing the survival of the plant. Advantageously, the nitrogen-deficient nutrient solution has a low electrical conductivity range extending between 0.4 to 1.2 mS/cm, preferably between 0.6 to 1 mS/cm. An example of such a composition may be a nutrient solution comprising N/P/K 0/10/15 (namely comprising 10% by weight of phosphore, e.g. in the form of P₂O₅ and 15% by weight of potassium in the form of K₂O).

Typically, the plant may be placed on trays with the aerial part of the plant above the tray and the root part below, the trays are placed on tables forming a retention area to collect the excess of a liquid diffused toward the plants, and the trays are transferred onto the tables at various stations. Teachings about this technology suitable for the aeroponic plant culture is more detailed in the international application WO2018054704A1 which is also incorporated by reference in the specification of the present invention. Advantageously, the solid/liquid extraction of the roots may comprise placing the roots in contact with an extraction solvent.

Advantageously, the above aeroponic culture conditions allow the obtention of some secondary metabolites, amongst which phenylpropanoid glycosides, in which at least one of the compounds among lavandulifolioside, verbascoside, leonoside A, leucosceptoside A and leonoside B is in amount superior (e.g. between 20% to 55%) compared to the amount of the same compounds obtained in soil-culture.

Without wishing to be bound by any theory, the above aeroponic or soilless culture conditions, in particular when performed with a stimulation step, may enable to promote the production of additional metabolites that may contribute to the overall biological effects of the extract.

Regardless of the process used to obtain it, the resulting root extract of *Leonurus japonicus* can be dissolved or diluted in an appropriate vehicle (or diluent solvent) to give a cosmetic ingredient. The cosmetic ingredient can be administered to the subject by topical route or incorporated into a cosmetic or a nutraceutical composition before use.

By "dilution solvent" or "vehicle" or "cosmetically acceptable vehicle" is meant a solvent used for diluting an already obtained root extract in liquid, solid or sticky form. The dilution solvent may be selected from alcohols, glycols, ethyl lactate, isopropyl myristate, triglycerides, tri-ethyl citrate, glyceryl isostearate, glyceryl stearate, or a mixture thereof. The diluent solvent may be selected from dipropylene glycol, propane-1,3-diol, propane-1,2-diol, pentylene glycol, butane-1,3-diol, glycerol or a combination of two or more thereof. Preferably, glycol may be propane-1,3-diol.

As detailed further below, another aspect of the invention relates to a cosmetic ingredient comprising an extract of the invention, and to a cosmetic or nutraceutical composition comprising an extract of the invention or a cosmetic ingredient of the invention.

### - Cosmetic use of the invention

An aspect of the present invention relates to the cosmetic use of a root extract of *Leonurus japonicus,* preferably as described above, as an active agent for ameliorating and/or maintaining the appearance and/or the quality and/or the comfort of a mature skin.

The invention also relates to the use of a root extract of *Leonurus japonicus,* preferably as described herein, as an active agent for preventing or treating a non-pathological alteration of the skin induced by hormonal decline in the perimenopause or menopause. More generally, the invention relates to the use of a root extract of Leonurus japonicus as an active cosmetic agent for delaying the onset, attenuating or alleviating hormonal skin aging, namely as a well-aging and/or well-being agent.

The use of the invention is preferably cosmetic, more preferably cosmetic and non-therapeutic.

As used herein, the term "cosmetic active ingredient or agent" refers to a compound exerting one or several cosmetic effects on the skin.

As used herein, "a cosmetic effect" refers to any non-therapeutic effect aiming at improving and/or modifying the appearance of the skin, the mechanical properties of the skin and/or the comfort of the skin. A cosmetic effect encompasses treating and/or correcting any non-pathological alterations of the skin or its appendages, and/or for alleviating the effects of the physiological, non-pathological biological events, such as a decline of the hormone levels, in particular those related to perimenopause or menopause on the skin or its appendages.

The root extract of *Leonurus japonicus* of the invention is typically for use in a woman having an average age of 40 or more.

The root extract of *Leonurus japonicus* of the invention is typically for use in a woman having a mature skin.

As used herein, "hormonal skin aging" refers to skin aging due to physiological hormonal, preferably estrogenic, decline or variation during perimenopause or menopause. Typically, "hormonal skin aging" refers to the physiological changes in the visual aspect and quality that can be observed in a mature skin as compared to a young skin (namely the skin of a woman before perimenopause).

As used herein, "a mature skin" refers to the skin of a woman having an average age of 40 or more, or a woman of at least 40 years old, preferably of at least 45 years old e.g., of at least 50 years old or of at least 55 years old. A mature skin refers to the skin of a woman showing one or more non-pathological alterations of the visual aspect and/or mechanical properties of the skin induced by hormonal decline.

In a particular embodiment, a mature skin refers to a skin that can show one or several signs of hormonal ageing in the skin such as decrease in sebum production, dryness, dehydration, decrease in collagen production, loss of elasticity, loss of firmness, slackening skin, dull complexion, fine lines, wrinkles, roughness and/or combinations thereof. The woman can also experiment a dry hair scalp and/or a decrease in hair density or thickness. A woman with mature skin can also experiment skin discomfort.

As used herein, "skin discomfort" refers to unpleasant skin sensations such as tightness, tingling, transient redness and transient flush. "Skin comfort" refers to a situation wherein the skin does not experiment such unpleasant skin sensations.

In some embodiments, a woman with mature skin is typically in perimenopause or in menopause.

In some embodiments, the extract of the invention is used in a woman in perimenopause. In some embodiments, the extract of the invention is used in a woman in menopause at most for 5 years.

Au used herein, "perimenopause" means "around menopause" and refers to refers to the time during which the woman body makes the natural transition to menopause, marking the end of the reproductive years. Perimenopause is also called the menopausal transition. Women can start perimenopause at different ages. Signs of progression toward menopause, such as menstrual irregularity (irregular periods in length and/or periodicity) and menopause-like symptoms such as hot flashes and sleep disorders, can be observed around 40 years old. During menopause, the level of estrogens rises and falls unevenly and a global decline is observed. Physiological estrogen decline observed in perimenopause and in menopause impairs the quality, the visual aspect and the comfort of the skin and can result in skin ageing.

Perimenopause period is considered as achieved and thus menopause established when the woman does not experiment any periods during twelve consecutive months.

By "skin" is meant any part of the skin of the human body, in particular the skin of the face, including lips, eyelids, the scalp, the skin of the hands, the legs and the feet. Preferably, the skin refers to the skin of the face, the skin of the hands and the hair scalp. The appendages of the skin include nails, body hair, eyebrows, eyelashes, and hair, preferably hair.

In preferred embodiments, the extract of the invention is applied topically, namely applied on a skin area.

Preferably, the skin area on which the extract of the invention is applied is healthy, which means that the skin does not show any skin disorders such as wounds, skin infection, eczema, or dermatitis.

By "non-pathological alteration of the visual aspect of the skin" is meant any non-pathological alteration of the skin appearance such as loss of radiance or glow, dull or non-uniform complexation, roughness, fine lines, wrinkles, tired aspect, and combination of two or more thereof. In the context of the invention, non-pathological alteration of the skin results from physiological hormone decline or variation observed in perimenopause or menopause.

By "skin quality" is meant the visual attributes of the skin such as tone, the radiance, the luminosity; the structural integrity and mechanical properties of the skin such as firmness, elasticity, density, resilience; the surface characters of the skin such as the texture; the functional properties of the skin such as barrier function, hydration level, sebum level, microcirculation, and cellular metabolism.

According to an embodiment, the use may be for regulating the production of sebum in the skin and/or for maintaining the skin barrier function and/or for improving the skin hydration.

According to an embodiment, the use may be for counteracting the decline of production of sebum lipids and/or for alleviating the skin dryness and/or for maintaining the skin hydration, and/or for maintaining the skin barrier function, more particularly in mature skin, e.g., in a woman in perimenopause or menopause.

By "regulating the production of sebum" is meant for increasing the production of lipids, in particular the sebum in skin, in dry skin, in particular but not limited to a mature skin under menopausal or perimenopausal conditions. Indeed, skin dryness may also occur in a young subject, namely a non-menopausal subject due to a disturbance of sebum production. Without wishing to be bound by any theory, the Applicant is of the opinion that the extract according to the invention may be able, on the one hand, to induce and/or enhance the sebogenesis in sebocytes in a mature skin, or in a subject presenting a dry skin due to the insufficiency of sebum. On the other hand, the extract of the present invention may not be able to increase and/or promote the production of sebum in the skin of a healthy subject, preferably a young subject less than 40 years of age.

In a particular embodiment, the invention relates to the use of a root extract of *Leonurus japonicus,* preferably as described herein, as a sebo-regulator agent in a woman in perimenopause or menopause experimenting a decrease in sebum production and/or skin dryness.

In this embodiment, a "sebo-regulator agent" refers to an agent able to increase and even restore the sebum production in the skin, including the scalp, of a woman in perimenopause or menopause to a level close to that observed prior to perimenopause or menopause.

According to an embodiment, the use may be for maintaining and/or improving the microvascular networks in the skin. Preferably, the use may be for improving the complexity and the density of the microvascular networks in the skin, more particularly in mature skin, e.g., in a woman in perimenopause or menopause.

In particular, the use may be for counteracting the vascular decline associated with aging, more particularly in a mature skin.

According to an embodiment, the use of the said extract may further be for promoting hair growth, for maintaining hair density, or a combination thereof. Advantageously, without wishing to be bound by any theory, the Applicant is of the opinion that ameliorating the microvascular networks in the scalp and in particular in the hair follicles may lead to a better oxygenation of this tissue, an improvement of nutrients delivery, and a support of cell homeostasis. The improvement of sebum production in the scalp can also contribute to hair quality and growth.

Without wishing to be bound by any theory, the Applicant believes that the dual effects of the extract of the invention on skin sebum production and microvasculature can improve the comfort of a mature skin e.g. in a woman in perimenopause and menopause.

In a particular aspect, the invention relates to the cosmetic use of a root extract of *Leonurus japonicus* for preventing or alleviating skin discomfort in a woman in perimenopause or menopause.

In a particular embodiment, the use of the invention is for preventing or alleviating dryness sensation, tightness, tingling, transient redness and transient flush.

In a particular embodiment, the use of the invention is for promoting skin well-being in a woman having an average age of 40 or more, or a woman of at least 40 years old, in perimenopause or menopause.

According to an embodiment, the use may be for potentializing and/or promoting the synthesis of pro-collagen I by human fibroblasts. Preferably, the use may for potentializing and/or promoting the synthesis of pro-collagen I by human fibroblasts in mature, e.g. in a woman in perimenopause or menopause.

According to an embodiment, the use may be for improving and/or ameliorating the visual appearance of signs of ageing by improving the mechanical properties of the skin, preferably a mature skin. Advantageously, the use may be for promoting and/or maintaining the elasticity, the firmness, the glow, the tone, the complexion, the density, the thickness, the barrier function, the hydration, the tone, the radiance of the skin, or a combination of two or more thereof.

Accordingly, the use may be for preventing or attenuating the formation of fine lines and wrinkles, dull complexion, or a combination thereof. Advantageously, the extract of the invention may be able to maintain the skin vitality by maintaining or restoring the integrity of the skin barrier function.

The root extract of *Leonurus japonicus* for use as described above, is preferably as defined in any embodiment described herein, in particular in the section entitled *"Leonurus japonicus* root extract of the invention".

According to a preferred embodiment, the root extract of *Leonurus japonicus* for use as described above is an alcoholic or an hydroalcoholic extract as described herein, such as a propanediol e.g., propane-1,3-diol root extract of *Leonurus japonicus.* In a preferred embodiment, said extract comprises lavandulifolioside. In a particular embodiment, the extract is a propane-1,3-diol extract. Preferably, lavandulifolioside accounts for more than 60% by weight of the total weight of the PPGs present in the extract. In some embodiments, lavandulifolioside accounts for more than 5% by weight relative to the total weight of the dry extract. In a particular embodiment, the extract is obtained from maceration of roots, preferably dried roots, of plants cultured in soilless conditions.

In a particular embodiment, the extract is enriched in phenylpropanoid glycosides. According to an embodiment, the root extract of *Leonurus japonicus* for use as described herein comprises phenylpropanoid glycosides selected from the group consisting of lavandulifolioside, verbascoside, leonoside A, leucosceptoside A and leonoside B, wherein the lavandulifolioside represents 5 to 30% by weight relative to the total weight of the dry extract.

It will be understood that the definitions and preferred embodiments made in the context of the (cosmetic) use of the root extract of *Leonurus japonicus* according to the above aspect of the invention *mutatis mutandis* apply to the root extract of *Leonurus japonicus* as described hereinafter. Particularly, the term "active ingredient", in the overall context of the present invention, may be understood in the broadest sense as the component that may exhibit the desired and intended aforementioned cosmetic effects, alone or may exhibit the activity together with one or more carriers that are themselves inactive.

The extract of the invention may be formulated into a cosmetic ingredient that can be e.g. incorporated into a cosmetic composition before being applied on the skin.

As mentioned above, in an additional aspect, the present invention relates to a cosmetic ingredient comprising a root extract of *Leonurus japonicus* as described herein in a cosmetically acceptable vehicle.

In an embodiment, the root extract of *Leonurus japonicus* may represent 0,0001 to 10% by weight, preferably 0.001 to 8% by weight, more preferably 0.01 to 7% by weight, in particular 0.1 to 5% by weight relative to the total weight of the cosmetic ingredient.

In an embodiment, cosmetically acceptable vehicle may be a solvent selected from the group of water, alcohols preferably C2-C5 alcohols, glycols or a combination of two or more thereof. C2-C5 alcohols may be preferably selected from the group of methanol, ethanol, propanol or a combination thereof. Glycols may be preferably selected from dipropylene glycol, propane-1,3-diol, propane-1,2-diol, pentylene glycol, butane-1,3-diol, glycerol or a combination of two or more thereof. More preferably, the solvent is propane-1,3-diol, preferably, a bio-based propane-1,3-diol. For instance, the cosmetically acceptable vehicle may be selected from tri-ethyl citrate, dipropylene glycol, propane-1,3-diol, propane-1,2-diol, pentylene glycol, butane-1,3-diol, glycerol, water or a combination of two or more thereof.

More preferably, the cosmetically acceptable vehicle is propane-1,3-diol, preferably, a bio-based propane-1,3-diol.

In some embodiment, the cosmetic ingredient can comprise an additional pharmaceutically acceptable excipient such as a preservative and/or a pH adjusting agent such as phytic acid or citric acid, preferably in an amount of less than 2%, such as less than 1% by weight e.g. from 0.1% to 1.0% by weight.

It will be understood that the definitions and preferred embodiments made in the context of the (cosmetic) use of the root extract of *Leonurus japonicus* according to the above aspect of the invention *mutatis mutandis* apply to the cosmetic ingredient comprising the root extract of *Leonurus japonicus.*

In some preferred embodiment, the root extract of the invention is topically applied on the skin in the form of a cosmetic composition.

In some other embodiment, the root extract of the invention is administered by oral route in the form of a nutraceutical composition.

The present invention also refers to a cosmetic or nutraceutical composition comprising a root extract of *Leonurus japonicus* according to the above aspect, or comprising a cosmetic ingredient comprising a root extract *of Leonurus japonicus* according to the above aspect, and optionally one or more excipients, which are preferably cosmetically or nutraceutically acceptable.

In some embodiment, the composition is a composition for topical use and is selected from the group consisting of a solution, a suspension, an emulsion, a cream, a paste, a gel, a lotion, a powder, a soap, a surfactant-containing water, an oil, a shampoo, and a spray.

In other embodiment, the composition is a nutraceutical composition which is administered orally, e.g., in the form of a tablet or a capsule filled with a powder.

According to a preferred embodiment of the composition, the root extract of *Leonurus japonicus* may represent 0.0001 to 15%, preferably 0.001 to 10%, more preferably 0.01 to 5% by weight, relative to the total weight of the cosmetic or nutraceutical composition. The modes of administration, the dosages and the optimal dosage forms of the cosmetic compositions according to the invention can be determined according to the criteria generally taken into account in the establishment of a cosmetic treatment adapted to a subject such as, for example, the type of skin. Depending on the type of administration desired, the cosmetic composition according to the invention may further comprise at least one cosmetically acceptable excipient.

The cosmetic composition according to the present invention may further comprise at least one adjuvant cosmetically known in the art, chosen from thickeners, preservatives, perfumes, dyes, chemical or mineral filters, moisturizing agents, thermal waters, etc. Accordingly, the cosmetic composition of the invention may further comprise other cosmetically active agents other than a root extract of *Leonurus japonicus* as defined above, such as other skin anti-aging agents (e.g., coenzyme Q10, plant extracts, peptides, etc.,), cleaning or purifying agents, anti-inflammatory agents, soothing agents, chemical or mineral sunscreens, etc.

Suitable cosmetic excipients are those known in the art. For example, suitable cosmetic excipients may be chosen from polymers, silicone compounds, surfactants, rheology agents, humectants, penetration agents, oily components, waxes, emulsifiers, film-forming agents and perfumes, electrolytes, pH adjusters, antioxidants, preservatives, dyes, pearlescent agents, pigments and mixtures thereof.

The cosmetic composition of the invention may comprise:
- from 0.005% to 10.0 % by weight of a root extract of Leonurus japonicus according to the invention,
- from 70 % to 99.995 % by weight of one or more cosmetically acceptable excipients, and,
- optionally, from 0.001% to 29.995% of one or more additional cosmetically active agents.

Accordingly, the cosmetic composition of the invention is advantageously intended for topical application.

The cosmetic composition of the invention is typically applied on the skin. The cosmetic composition may be in any form suitable for such a topical administration. In a particular embodiment, the cosmetic composition of the Invention is selected from the group consisting of aqueous solutions, hydroalcoholic solutions, oil-in-water emulsions (O/W) or water-in-oil (W/O) or multiple), including nanoemulsions, aqueous gels (also called hydrogels), suspensions, preferably in aqueous or hydroalcoholic medium, liposome suspensions, oily compositions, serums, powders, lotions, milks, creams, ointments, gels, foams, balms, foams, and aerosols.

It may, in particular, be in the form of a cream, a milk, a lotion, a gel, a serum, a spray, a mousse, a solution, an ointment, an emulsion, a patch or a mask.

More generally, the composition according to the invention can also take the form of any cosmetic product. It may be a skin care product, a make-up product or a body hygiene product, for example a lotion, a milk, a serum, an aqueous or oily gel, an emulsion, a cream, a gel-cream, an ointment, a balm, foundation, spray, stick, lipstick, gloss, nourishing mask, shower gel, cleansing oil, soap, shampoo or conditioner, hair mask or oil including massage oil.

Suitable nutraceutical excipients are known in the art. Examples may include water soluble polymers like cellulosic polymers, acrylate polymers and copolymers, polyvinylpyrrolidones, water soluble polyethylene glycols, vinyl copolymers. The nutraceutical composition may be for example a food supplement in the form of a solid, coated or non-coated tablet, liquid, powder, soft or hard gelatin capsule, etc.

It will be understood that the definitions and preferred embodiments made in the context of the (cosmetic) use of the root extract of *Leonurus japonicus* according to an aspect of the invention *mutatis mutandis* apply to a composition comprising said root extract.

The present invention also refers to a cosmetic non-therapeutic method for ameliorating and/or maintaining the quality and/or the comfort of a mature skin, the method comprising a topical application of an effective amount of a root extract of *Leonurus japonicus* as described herein, in particular an alcoholic and/or a hydroalcoholic root extract of *Leonurus japonicus* according to the above aspect, or a cosmetic ingredient according to the above aspect on the part of the body of a subject in need.

As mentioned above, the subject may be a woman of at least 40 years old and having mature skin, in particular a woman of at least 40 years old in perimenopause or menopause.

According to an embodiment, the method may be for regulating the production of sebum in the skin and/or for maintaining the skin barrier function and/or for improving the skin hydration.

According to an embodiment, the method may be for maintaining and/or improving the microvascular networks in the skin.

In the cosmetic methods and uses as described herein, the dose to be administered and the frequency of administration according to the invention vary according to the cosmetic effect sought, the characteristics of the individual. Typically, the extract of the invention, or the cosmetic composition comprising it, can be applied to a skin area to be treated once or twice daily, e.g. after skin cleaning, for several consecutive weeks or even months, for example for at least 3 months. For example, the subject can apply a dose of 1 g to 2 g of cosmetic composition on the face in the morning and/or in the evening. Preferably, the extract or the cosmetic composition of the invention is applied on the skin by massage. The application by massage may last from a few seconds to several minutes, e.g. from 1 min to 30 min.

It will be understood that the definitions and preferred embodiments made in the context of the (cosmetic) use of a root extract of the plant *Leonurus japonicus* according to an aspect of the invention *mutatis mutandis* apply to the non-therapeutic method according to the other aspect of the present invention.

### Examples

### Example 1: Method of quantification of phenylpropanoid glycosides

All samples were analyzed using the UHPLC Shimadzu Nexera X2 system (Shimadzu, Japan) with a PDA detector coupled to a mass spectrometer LCMS2020 (electrospray ionization in a negative ion mode, *m*/*z* 100-1000), using a Kinetex EVO C18 reverse phase column (150 mm x 2.1 mm, 2.6 µm, Phenomenex, USA), maintained at 40°C during all analyses. The mobile phase was composed of ultrapure water (Mili-Q, Merck Millipore) + 0.1 % of formic acid (Carlo Erba, France) (phase A) and pure acetonitrile (Sigma-Aldrich Chemie, Germany) (phase B), delivered at 0.5 ml/min with the gradient of the phase B as follows: 5->15% (0->0.3 min) ; 15->18% (0.3->3min), 18->40% (3->6.5min), 40->95% (6.5->6.6min), 95% (6.6->8min), 95->5% (8->8.10 min), 5% (8.10->10.60 min)

In the context of the present disclosure, the phenylpropanoid glycosides - hereinafter "PPGs" - designate the sum of five compounds of these families identified in *Leonurus japonicus* root extract of the present invention, hereinafter "LJRE". The five compounds considered are the followings (described in increasing order of elution using UHPLC analysis as described above): lavandulifolioside (LAV), verbascoside (VER), leonoside A (LEO-A), leucosceptoside A (LEU-A) and leonoside B (LEO-B). Content in PPGs is the sum of the mass concentrations of the five compounds considered.

The quantification of PPGs was carried out using the well-known verbascoside standard, which was prepared at the concentration of 100 mg/L in an DMSO/water mixture at 70/30 volume/volume (v/v). The content in PPG was expressed in equivalent of verbascoside in each extract sample.

In the context of the present disclosure, the concentration of LAV, VER, LEO-A, LEU-A and LEO-B in the extracts of *Leonurus japonicus* expressed in mmol/L, was determined by measuring the areas of the peaks corresponding to LAV, VER, LEO-A, LEU-A and LEO-B on HPLC chromatogram (at 330 nm) of said extract. LAV, VER, LEO-A, LEU-A and LEO-B peak areas were then divided by the peak area of the standard solution comprising 0.160 mmol/L of verbascoside and multiplied by molar concentration of verbascoside standard (0.160 mmol/L). The values of molar concentration of LAV, VER, LEO-A, LEU-A and LEO-B (expressed in mol/L) were converted to the mass concentrations (expressed in g/L) by multiplying them by the molecular weights of both compounds.

The quantification of LAV, VER, LEO-A, LEU-A and LEO-B was conducted according to the following equations: $\frac{{Conc}_{PPGx}^{Y} \left[\frac{mol}{L}\right] = DF ⋅ \left({Conc}_{VER - STD}^{STD}\left[\frac{mol}{L}\right]\right) ⋅ \frac{{A}_{PPGx}^{Y} at 330 nm}{{A}_{VER - STD}^{STD} at 330 nm}}{{Conc}_{PPGx}^{Y} \left[\frac{g}{kg}\right] = \frac{{ Conc}_{PPGx}^{Y} \left[\frac{mol}{L}\right] ⋅ {MW}_{PPGx} \left[\frac{g}{mol}\right]}{{d}_{L} \left[\frac{kg}{L}\right]}}$ where:
PPGs - phenylpropanoid glycosides;
DF - dilution factor;
${Conc}_{PPGx}^{Y}$ - concentration of PPG X (LAV, VER, LEO-A, LEU-A and LEO-B) in a sample Y;
${Conc}_{VER}^{STD}$ - concentration of the standard solution of verbascoside (0.160 mmol/L);
${A}_{PPGx}^{Y}$ - peak area of PPG X (LAV, VER, LEO-A, LEU-A, LEO-B) in a sample Y at 330 nm ;
${A}_{VER - STD}^{STD}$ - peak area of verbascoside standard at 330 nm, corresponding to the concentration of the standard solution at 100 mg/L = 0.160 mmol/L;
MW_{PPGx}- molecular weight of PPG X (LAV : 756 g/mol ; VER : 624 g/mol ; LEO-A : 770 g/mol ; LEU-A : 638 g/mol ; LEO-B : 784 g/mol);
d_{L} : density of *Leonurus japonicus* root extract (1,052 kg/L).

Content in PPGs is the sum of mass concentrations of the five compounds considered as described in the following formula: ${Conc}_{PPG}^{Y} \left[\frac{g}{kg}\right] = {\sum }_{x = 1 to 5} {Conc}_{PPGx}^{Y} \left[\frac{g}{kg}\right]$

### Example 2: Culture of Leonurus japonicus using different culture media to maximize PPGs content in the roots

Root extracts as described below were prepared from plants whose commercial seeds had been purchased from a French provider (Conservatoire National des Plantes à Parfum, Médicinales et Aromatiques, CNPMAI) in 2016, 2020 and 2021. From 2022, the *Leonurus japonicus* plants were preserved and multiplied by the Applicant.

*Leonurus japonicus* plants were cultivated aeroponically for a maximum of four weeks using a nutrient solution comprising, by weight, 15% of nitrogen, 10% of phosphorus and 30% of potassium - i.e., N/P/K at 15/10/30 - with different durations (i.e., 1 to 4 weeks) for each medium to evaluate the best condition to maximize PPG content in roots. Conditions include a cultivation period with a stimulating step by means of a nitrogen-deficient nutrient solution at 0/15/40. Roots have been extracted via DMSO/water (70/30 v/v). The HPLC-UV-MS analysis according to Example 1 demonstrates that all the PPGs were strongly increased by a factor of 2.6 for aeroponics roots under stimulation conditions.

**Table 1: Concentrations of LAV, VER, LEO-A, LEO-B, and PPG in different roots of Leonurus japonicus cultivated in different conditions (n=2). No stimulation means nutrient solution of N/PK at 15/10/30 for 4 weeks. Weak stimulation means nutrient solution N/PK at 15/10/30 for 3 weeks, followed by nutrient solution N/PK at 0/10/30 for 1 week. Strong stimulation means nutrient solution N/PK at 15/10/30 for 1 week, followed by N/PK at 0/10/30 for 3 weeks.**

| | LAV (mg/L) | VER (mg/L) | LEO-A (mg/L) | LEO-B (mg/L) | PPG (mg/L) |
|---|---|---|---|---|---|
| No stimulation | 227 | 47 | 45 | 31 | 419 |
| Weak stimulation | 558 | 82 | 86 | 53 | 838 |
| Strong stimulation | 688 | 94 | 123 | 86 | 1106 |

### Example 3: Phytochemical analysis of PPGs content in different tissues of Leonurus japonicus with different modes of cultivation

For this example, the term "cultivating in soil" is meant cultivating the plant in field or pot grown under greenhouse.

*Leonurus japonicus* plants were sowed and multiplied. After five weeks of culture in soil under a greenhouse, plants were dispatched into two groups: i) some plants were maintained in cultivation in soil and ii) the others were cultivated in aeroponics. *Leonurus japonicus* plants were cultivated aeroponically for 2 weeks with a culture medium of N/P/K at 15/10/30 and then with a nitrogen-deficient solution of N/P/K at 0/15/40 for a 4-weeks.

Plants in aeroponics and in soil were harvested the same day to compare PPGs content in roots and in aerial parts. Roots and aerial parts were cut, harvested, and dried. For the preparation of each extract, 15 mg of ground roots in powder form or 15 mg of ground aerial parts in powder were macerated in 1.5 mL in the reference extraction solvent made of DMSO/water (70/30 v/v) with stirring at ambient temperature for 1h. The samples were centrifuged and analyzed by HPLC-UV-MS according to Example 1.

**Table 2: Content in PPGs expressed in % of phenylpropanoid glycosides weight per weight of dry root or aerial matter in different extracts of root or aerial parts of Leonurus japonicus in different culture conditions (n=2)**

| | PPGs content (%) |
|---|---|
| Aerial parts of plant in aeroponics | 0.31 |
| Aerial parts of plants in soil | 0 |
| Roots of plants in aeroponics | 5.25 |
| Roots of plants in soil | 3.00 |

Surprisingly, no PPGs were detected in the aerial parts of plants cultivated in soil compared to those cultivated aeroponically. It was observed that the extract of the roots of *Leonurus japonicus* cultivated in aeroponics and in soil both contain PPGs, but in different quantities, namely 5.25% and 3.00% respectively.

This study also confirmed that:
- roots from *Leonurus japonicus* contain much more PPGs than aerial parts, whether they are cultivated in soil or in aeroponic conditions,
- for plants in aeroponic, the PPGs content is higher for roots than for aerial parts. The content increases by a factor of 1590%.
- the PPG root content is higher in aeroponic cultivation than in soil cultivation by a factor of 48%.

For optimizing the extraction yield of PPG, the following extraction solvents have been chosen for the solid/liquid extraction by maceration: DMSO/water at 70/30 (v/v), pure water, a mixture of propane-1,3-diol/water at 50/50 (v/v), pure propane-1,3-diol, and a mixture of glycerin/water at 70/30 (v/v), the extraction process being conducted at 25°C or at 50°C for 30 min to 48 hours. It is observed that the pure propane-1,3-diol showed the better extraction yield, namely 4.38% to 5.11% by weight of PPG over total weight of the dry extract, compared to DMSO/water at 70/30 (v/v) (3.17% to 3.89% by weight of PPG over total weight of the dry extract).

In this regard, a root of an aeroponic cultivated *Leonurus japonicus,* stimulated with a nitrogen-deficient nutrient solution i.e., N/P/K at 0/10/15 and extracted with pure propane-1,3-diol presents the following general characteristics:
- dry extract content: 14 to 25 g/Kg
- PPGs: 1900 to 4900 mg/Kg (i.e. 7.6% to 35.0% of the dry extract)
- Lavandulifolioside: 1600 to 3700 mg/Kg (i.e. 6.4% to 26.4% of the dry extract)
- Verbascoside: 70 to 450 mg/Kg (i.e. 0.3% to 3.2% of the dry extract)
- Leonoside A: 90 to 350 mg/Kg (i.e. 0.36% to 2.5% of the dry extract)
- Leucosceptoside A: 14 to 25 mg/Kg (i.e. 0.06% to 0.18% of the dry extract)
- Leonoside B: 70 to 150 mg/Kg (i.e. 0.3% to 1.1% of the dry extract)

Such an extract can be diluted in a dilution solvent for constituting the cosmetic ingredient. Furthermore, a commercial dried root of *Leonurus japonicus* (hereinafter so as cLJRE, purchased on July 2020 (Xian Dichuang Biotechnology Co. Ltd) has also been extracted with a propane-1,3-diol in the same conditions as those for the extraction of the roots cultivated in aeroponics conditions according to the invention as described above. It was confirmed that the cLJRE contains less than 3.00% by weight of PPGs relative to the total weight of the dry extract.

For the biological experiments here below, it has been weighed 10 g of a dry extract of LJRE as obtained in Example 3 (namely an extract obtained from stimulated roots of aeroponic cultivated *Leonurus japonicus* obtained by extraction with propane-1,3-diol has been solubilized in 99 g of propane-1,3-diol as a dilution solvent, i.e., 1% of dry extract of LJRE in 99% of propane-1,3-diol for constituting the cosmetic ingredient. Particularly, the 10 g of dry extract contains 1.5 g of total PPGs, i.e., 15% by weight of PPGs over the total weight of the dry extract. The lavandulifolioside represents about 80% of the total PPGs i.e., 1.2 g/kg of the cosmetic ingredient.

### Example 4: in vitro experiments

### 4.1. LJRE induces sebogenesis in menopausal conditions

**Figures 1** highlights the abilities of LJRE to induce lipid production by human sebocytes under menopausal stress.

Sebaceous glands, via sebocytes, produce sebum essential for maintaining skin hydration and barrier integrity. Under normal physiological conditions, sebum prevents skin dryness by forming a protective lipid layer. Sebum production is hormonally regulated, and during menopause, the decline in estrogen reduces sebaceous gland activity and lipid synthesis, leading to decreased sebum levels and exacerbated skin dryness.

### Methods:

In hormonal aging study model, human primary sebocytes were incubated under menopausal conditions with significantly reduced levels of key hormones (same menopausal conditions as described in Example 4, Section 4.3: Methods) and treated or not by LJRE at 0.1% for 48 hours. Additionally, treatment with non-menopausal level of hormones was used as positive control. Lipid droplets were stained with Bodipy dye and nucleus were stained using Hoechst reagent. Total lipid content was quantified by calculating the ratio of Bodipy staining area on nucleus number and results were expressed in percentage of variation compared to control condition.

### Results:

In menopausal conditions, lipid production was significantly reduced by 70% compared to non-menopausal level. This result was expected according to scientific literature data and validated the study model. It was observed that LJRE also allows to significantly counteract menopausal stress by inducing lipid production by 89%. It was demonstrated that LJRE significantly enhances sebogenesis under menopausal conditions. Therefore, LJRE can be a cosmetic ingredient effective for addressing hormonal aging and the specific needs of menopausal women, helping to counteract the decline in sebum production and alleviate associated skin dryness, particularly in a mature skin.

### 4.2. LJRE improves microvascular network

**Figure 2** highlights the abilities of LJRE to improve microvascular network formation under menopausal conditions.

The skin microvasculature network is essential for maintaining tissue oxygenation, nutrient delivery, and supports dermal homeostasis. At the onset of menopause, the significant reduction circulating estrogen levels also disrupts angiogenic processes, resulting in decreased microvascular density, altered network architecture, and uneven skin tone.

### Methods:

Pool of human umbilical vein endothelial cells (HUVEC) from 10 donors were seeded into a matrigel matrix and treated or not by LJRE at 0.1% and 0.5% in addition of hormonal concentration of menopausal women (same menopausal conditions as described in Example 4, Section 4.3: Methods). In a positive control, certain cells were treated with hormonal concentration on non-menopausal (NM) conditions. Cells were incubated for 16h to allow microvascular formation. Microvascular network formation was characterized by image analysis using an ImageJ plug-in. The number of master junctions (junctions that link at least three segments) and meshes (areas enclosed by segments) were calculated by the software and used as parameters to analyze the network. Results are expressed in percentage of variation compared to untreated control condition.

### Results:

Under menopausal conditions, LJRE significantly enhanced microvascular network formation. LJRE at 0.05% was able to restore the master junctions and mesh numbers to levels comparable with the non-menopausal condition. This pattern reflects a denser, more complex vascular architecture with increased ramification, suggesting the formation of a more functional and mature microvascular network. It demonstrated a substantial increase in microvascular network formation, up to +138% in junctions' formation and +85% in number of meshes. Notably, these improvements showed comparable efficacy with LJRE with no significant differences at equivalent concentrations on the three parameters. Therefore, it was demonstrated that LJRE improves the complexity and density of the microvascular network. This improvement supports optimal vascular function, promoting better oxygenation and nutrient delivery. Such effects are particularly valuable and highly expected for enhancing overall skin quality and counteracting the vascular decline associated with menopause, helping to maintain skin vitality and radiance, in particular mature skin.

### 4.3. LJRE counteracts menopause effect on collagen metabolism

**Figure 3** highlights the abilities of LJRE to potentialize the synthesis of pro-collagen I by NHDF under menopausal stress.

Collagen, particularly type I collagen, is a major structural protein synthesized as procollagen by dermal fibroblasts, playing a crucial role in skin elasticity and firmness. During menopause, the decline in hormones levels disrupts fibroblast function, significantly reducing procollagen I production. This hormonal shift leads to decreased collagen content and increased matrix degradation, accelerating skin aging and resulting in reduced dermal thickness and elasticity.

### Methods:

The study model aimed to simulate hormonal aging by reducing key hormone concentrations to mimic post-menopausal serum levels. NHDF from a 19-year-old woman were initially grown to confluence, then stimulated with non-menopausal hormone levels to enhance extracellular matrix production with collagen composition close to that of young women's dermis. This was followed by a serum deprivation phase to stabilize cell activity for 24h. Finally, cells were incubated under menopausal conditions with significantly reduced levels of key hormones and growth factors (17β-estradiol, progesterone, dehydroepiandrosterone, growth hormone and insulin-like growth factor) and treated or not by LJRE at 0.1% for 72 hours. Additionally, treatment with non-menopausal level of hormones was used as positive control. Pro-collagen I production was measured by ELISA and total proteins were quantified using Bradford assay. Analysis was done by calculating the ratio of pro-collagen I on total proteins and results were expressed in percentage of variation compared to menopausal control condition.

### Results:

Drop of hormone levels in menopausal conditions, significantly decreased pro-collagen I production by 30% compared with non-menopausal conditions. These experimental results are consistent with what the scientific literature described and, hence, validated the study model. The obtained results showed that LJRE allows to significantly induce pro-collagen I production by 57% under menopausal stress, recovering to non-menopausal (NM) state (no significant difference with the NM condition). It can be concluded that LJRE demonstrated a significantly greater efficacy in stimulating pro-collagen I production in menopausal conditions. The results indicate that the LJRE is able to mitigate collagen loss under menopausal stress, highlighting their effectiveness for a use in cosmetic products targeting mature skin.

### Example 5: cosmetic composition containing LJRE

| **Composition of gel cream** | **Component in %** |
|---|---|
| Water | 86.39 |
| LJRE in propane-1,3-diol | 1.00 |
| Citric acid, sodium citrate | 1.00 |
| Ammonium acryloyldimethyltaurate/VP crosspolymer | 0.50 |
| Cetearyl alcohol | 2.00 |
| Glyceryl stearate SE | 3.00 |
| Phenoxyethanol (and) Methylparaben (and) Ethylparaben | 1.10 |
| Isodecyl neopentanoate | 5.00 |
| Fragrance | 0.01 |

## Claims

1. A cosmetic use of an alcoholic or hydroalcoholic root extract of *Leonurus japonicus,* as an active ingredient, for ameliorating and/or maintaining the appearance and/or the quality and/or the comfort of a mature skin.

2. The cosmetic use of claim 1, wherein the extract is applied on the skin of a woman having an average age of 40 or more, in perimenopause or menopause.

3. The cosmetic use according to claim 1 or 2, wherein the use is for regulating the production of sebum in the skin and/or for maintaining the skin barrier function and/or for improving the skin hydration.

4. The cosmetic use according to any one of claims 1 to 3, wherein the use is for maintaining and/or improving the microvascular networks in the skin.

5. The cosmetic use according to any one of claims 1 to 4, wherein the use is for promoting skin complexion, skin glow, skin tone, skin radiance or a combination of two or more thereof.

6. The cosmetic use according to any one of claims 1 to 5, wherein the use is for alleviating or preventing skin discomfort, in particular alleviating or preventing dryness sensation, tightness, tingling, transient redness and transient flush.

7. The cosmetic use according to any one of claims 1 to 6, wherein the use is for potentializing the synthesis of pro-collagen I.

8. The cosmetic use according to any one of claims 1 to 7, wherein the use is for preventing or attenuating the formation of fine lines and wrinkles.

9. The cosmetic use according to any one of claims 1 to 8, wherein the extract is a propane-1,3-diol extract.

10. The cosmetic use according to any one of claims 1 to 9, wherein the extract comprises phenylpropanoid glycosides selected from the group consisting of lavandulifolioside, verbascoside, leonoside A, leucosceptoside A and leonoside B, wherein the lavandulifolioside represents 5 to 30% by weight relative to the total weight of the dry extract.

11. A root extract of *Leonurus japonicus* comprising phenylpropanoid glycosides selected from the group consisting of lavandulifolioside, verbascoside, leonoside A, leucosceptoside A and leonoside B, wherein the lavandulifolioside represents 5 to 30% by weight relative to the total weight of the dry extract.

12. A root extract according to claim 11, wherein the total amount of phenylpropanoid glycosides represents 7.5% to 36% by weight, preferably 8% to 30% by weight, more preferably 10% to 30% by weight, even more preferably 12% to 30% by weight, in particular 12% to 25% by weight, relative to the total weight of the dry extract.

13. A cosmetic ingredient comprising a root extract of *Leonurus japonicus* as defined in any one of claims 11 or 12 in a cosmetically acceptable vehicle.

14. Cosmetic ingredient according to claim 13, wherein the cosmetically acceptable vehicle is a solvent selected from the group of tri-ethyl citrate, dipropylene glycol, propane-1,3-diol, propane-1,2-diol, pentylene glycol, butane-1,3-diol, glycerol, water or a combination of two or more thereof, preferably propane-1,3-diol.

15. Cosmetic composition comprising a root extract of *Leonurus japonicus* as defined in claim 11 or 12, or comprising a cosmetic ingredient as defined in claim 13 or 14, and optionally one or more excipients which are preferably cosmetically acceptable, wherein the composition is a composition for topical use selected from the group consisting of a solution, a suspension, an emulsion, a cream, a paste, a gel, a lotion, a powder, a soap, a surfactant-containing water, an oil, a shampoo, and a spray.

16. A cosmetic non-therapeutic method for ameliorating and/or maintaining the quality and/or the comfort of a mature skin, the method comprising a topical application of an effective amount of an alcoholic or a hydroalcoholic root extract of *Leonurus japonicus* preferably as defined in claim 11 or 12, or a cosmetic ingredient as defined in claim 13 or 14 on a part of the skin of a subject in need thereof.
